# EUROPEAN PATENT APPLICATION

(11) **EP 4 325 513 A1**
(43) Date of publication of application: **21.02.2024**
(21) Application number: 22190975.7
(22) Date of filing: 18.08.2022
(51) Int. Cl.: G16H 30/40

(54) **DISTRIBUTED MEDICAL IMAGE PROCESSING**

(71) Applicant: Siemens Healthineers AG, 91301 Forchheim (DE)
(72) Inventor: Campagna, Swen, 91238 Engelthal (DE); Huang, Yan Tu, Shenzhen, 518000 (CN); Stranjak, Armin, 91080 Uttenreuth (DE)
(74) Representative: Siemens Healthineers Patent Attorneys

(57) **Abstract**

A method of performing a medical imaging process is described. The method comprises the step of dividing a computing task (T) of a medical imaging system unit (1) into a set (S) of sub-tasks (T₁, ..., Tₙ), wherein the computing task (T) is related to a generation of medical image data based on measurement data. The method also comprises the step of selecting at least one sub-task (Tₖ) of the set (S) of sub-tasks (T₁, ..., Tₙ) for external execution. Further, the method includes the step of exchanging a sequence (S-TDS) of signal including task delegation signals (TDS) with a plurality of remote computing units (2), preferably via a distribution (3) unit for selecting at least one of the remote computing units (2) for execution of the selected at least one sub-task (Tₖ), whereby the sequence (S-TDS) of signals comprises at least one request signal (RS) sent by the medical imaging system unit (1). The method also comprises the step of outsourcing the at least one selected sub-task (Tₖ) to the selected remote computing unit (2) for remotely generating a computing result (CRₖ) of the at least one selected sub-task (Tₖ). The method also comprises the step of receiving the generated computing result (CRₖ) from the selected remote computing unit (2) and also the step of completing the computing task (T) with the computing result (CRₖ) received from the selected remote computing unit (2). Further, some sort of award or compensation is awarded as a result of successful task execution to the selected remote computing unit (2). Further, a method for executing a sub-task (Tₖ) of a remote medical imaging system unit (1) is described. Furthermore, a method for distributed executing a medical imaging process of a medical imaging system (10) is described. Besides, a medical imaging system unit (1) is described. Beyond, a computing unit (2) for performing a sub-task (Tₖ) of a remote medical imaging system unit (1) is described. Moreover, a medical imaging network is described.

## Description

The invention relates to a method of performing a medical imaging process. Further, the invention is directed to a method of performing a sub-task of a remote medical imaging system unit. Furthermore, the invention concerns a method of distributed performing of a medical imaging process of a medical imaging system. The invention also regards a medical imaging system unit. Besides, the invention pertains a computing unit for performing a sub-task of a remote medical imaging system unit. The invention also applies to a medical imaging network.

Medical imaging processes (for example MR imaging processes) depict the inside of the body of a patient, either in full or in part. Imaging techniques help doctors to diagnose a disease, to assess its severity and to monitor sick patients. Most imaging procedures are painless, relatively safe and non-invasive. Medical imaging includes, for example, imaging techniques based on radiation, ultrasound scans and magnetic resonance imaging (abbreviated as MRI or MR imaging).

Imaging modalities, in particular magnetic resonance imaging systems (abbreviated by MRI system or MR system), need substantial computing power for quickly performing preparation, execution and evaluation, in particular a reconstruction of images based on raw data, of an imaging process. It is important that reconstructed images are available to the operator of a scan unit shortly after executing the measurement, i.e. the acquisition of raw data. Short time of an image reconstruction is crucial, in particular in emergency circumstances where patient's health could be at risk.

For a magnetic resonance imaging system, two different approaches are conventionally used for achieving a sufficient speed of reconstruction. The first is to equip a localized processing: This approach encompasses a task processing on a localised computer system that is normally in a close vicinity of devices that produce images, typically MR or CT scanners, or any other medical imaging device. Such solution is relatively effective but not scalable and highly expensive. With the development of modern imaging devices, it cannot fulfil ever-growing requirements for increased computational power, for example due to a rise of imaging resolution or the number of receiving channels of an MR signal or an introduction of new compute intensive applications like parallel image or compressed sensing.

The second approach includes an offloading of the computing task, especially the image reconstruction, somewhere else, for example somewhere in the cloud, i.e. in a cloud computing system. This approach would solve challenges of localised processing by providing an infrastructure to scale image processing by distribution of processing tasks remotely. However, such cloud-based processing would be monolithic in nature, the tasks would not be compartmentalised of divided into smaller chunks. The tasks can naturally be big in size, in terms of processing time and resources like memory of CPU power, and therefore finding an appropriate computer system capable to execute such complex task could be a challenge. Furthermore, a bandwidth can also be a problem in transporting huge task results at once between cloud nodes.

In order to tackle challenges concerning to the second approach, a method that would divide tasks into smaller chunks in order to make the processing tasks more independent in terms of the requirements for high computing power, both locally and remotely, would be satisfactory ones. In this way, processing power requirements are not so stringent and even smaller computing systems could participate in such processing activities, since tasks are divided in smaller chunks in order to be manageable by the systems with less computing power.

However, another challenge emerges as the result of the complexity of task distribution in an open and competitive environment. If we imagine cloud-based computing resources, distributed on the network with heterogeneous processing capabilities, it is obvious that such task distribution would require systematic and decentralized coordination between these resources and tasks in order to achieve efficiency and effectiveness of such task execution within given time and processing costs.

Hence, there is a problem of an efficient organization of a distributed execution of a medical imaging task.

The before-mentioned problem is solved by a method of performing a medical imaging process according to claim 1, a method of performing a sub-task of a remote medical imaging system unit according to claim 11, a method of distributed performing a medical imaging process of a medical imaging system according to claim 13, a medical imaging system unit according to claim 14, a computing unit for performing a sub-task of a remote medical imaging system unit according to claim 15 and a medical imaging network according to claim 16.

In the method of performing a medical imaging process, a computing task of a medical imaging system unit, also named as requestor, is divided into a set of possibly consecutive or partially parallel sub-tasks. The computing task is related to a generation of medical image data based on measurement data. At least one of the sub-tasks of the set of sub-tasks are selected for external execution preferably by a remote computing unit which is not part of the "own" medical imaging system. In most cases, a remote computing unit, also named as executor, is spatially separated from the "own" medical imaging system. Hence, the remote computing unit provides a kind of external support to the medical imaging system unit. The medical imaging system unit comprises technical means for performing a computing task related to the medical imaging process of a medical imaging system. As later described in details, such a computing task may comprise calculations for realizing a medical imaging process, in particular a generation of protocol data, wherein the protocol data are intended to define a process for generating measurement data. Further, such a computing task may comprise a generation of reconstructed image data based on the measurement data. While the medical imaging system unit is a part of a medical imaging system, the remote computing unit may be a part of a remote medical imaging system, however, the remote computing unit may also be a compute only unit, i.e. a computer which is preferably separated from a medical imaging system and which offers its computing service to medical imaging systems, in particular medical imaging system units.

Further, a sequence, i.e. a coupling sequence of signals, comprising task delegation signals, is exchanged with a plurality of remote computing units, preferably via a distribution unit, for selecting at least one of the currently freely available remote computing units for execution of the selected sub-task. The sequence of task delegation signals comprises at least one request signal, preferably comprising a request for executing the selected sub-task sent by the medical imaging system unit for execution of the selected sub-task.

For finding a remote computing unit for executing the selected sub-task, a sequence of signals is exchanged. The sequence comprises task delegation signals exchanged with a plurality of remote computing units. The sequence of task delegation signals implements a dialogue between at least one medical imaging system unit and a plurality of remote computing units for arranging a coordinated performing of the task, in particular for delegating the execution of the selected sub-task to one of the remote computing units. The sequence comprises the above-mentioned request for executing the selected sub-task and preferably and further, as an answer to such a request, detailed information about performance and resources which the remote computing units are enabled to provide for execution of the sub-task. The signals are exchanged preferably via a distribution unit with the remote computing units. The communication is performed for selecting at least one of the remote computing units for execution of the selected at least one sub-task. Thereby, the sequence of task delegation signals comprises at least one request signal sent by the medical imaging system unit. The request signal comprises a request for executing the selected sub-task. As later described in detail, the communication for selecting an available appropriate remote computing unit for execution of the selected sub-task is preferably realized by an auction-like negotiation process. Such a negotiation process is preferably based on second best estimation auction, also named "Vickrey auction", since by using such an auction type, the offers of the remote computing units are more realistic and encourage a truthful task execution estimation. Such an auction-like bidding process enables a selection of an optimal remote computing unit based on an amount of the resources for compensation of the remote execution of the sub-task available for the medical imaging system unit.

The distribution unit comprises a computer network (intranet or internet, preferably based on TCP/IP protocol. The distribution unit enables an inclusion of a plurality of medical imaging system units, also named requestors, and a plurality of remote computing units, also named executors, into the computer network. Such an integration is simply a consequence of such internet protocol where every requestor holds a list of all available executors (via third party application or directly) with which they communicate. As soon as a new executor appears or disappears from the network, the requestors, on demand, can refresh their lists. This can happen on demand (manually) or automatically via third party.

The distribution unit implements a kind of "marketplace" which comprises an environment where interesting parties can offer their services and the other parties can ask for these services. It is normally many-to-many relationship where a requestor can contact many executors and an executor can be contacted by many requestors. Communication is normally one-to-one between any requestor and any executor but as said, a requestor maintains several of these communication channels with many executors. It is called "marketplace" as it is similar to "a market" where exchange of services happens for a certain compensation (financial or exchange of available resources). Technologies or platforms like Web Services, a .NET distributed platform using a Communication Framework, Micro-services or SDLMAS etc. can be used for implementing such a distribution network.

After selecting the remote computing unit for performing the selected sub-task, the at least one selected sub-task is outsourced to the selected remote computing unit for remotely generating a computing result of the at least one selected sub-task.

Further, the generated computing result is received from the selected remote computing unit.

After that, the computing task is completed with the computing result received from the selected remote computing unit.

The computing task is related to the generation of image data based on measurement data and preferably to a control task for controlling an imaging process or a task for processing measurement data for generating image data. In particular, the computing task is related to the generation of control data to be transmitted to a scan unit and/or to the processing of raw data received from the scan unit. As later discussed in detail, the computing task related to the process of generating image data preferably includes a reconstruction task or a filtering task.

In particular, a complicated reconstruction task, for example a parallel reconstruction, may divided into sub-tasks.

Such a complicated reconstruction task may include one of the following reconstruction types:
Parallel reconstruction may be applied to an acquisition of different images in parallel (e.g. by interleaving k-space lines of different images) or by a computationally parallel image reconstruction of different images for whatever reason (e.g. because of a 3D-Fast Fourier Transformation or because raw-data flows in so rapidly), while the reconstruction of a single image is very computationally intensive and thus different images may be reconstructed in parallel then.

A complicated reconstruction task may also comprise an MRI "parallel imaging" which is an acquisition technique to generate an image by reducing the number of acquired k-space lines (by the use of multiple receive channels). Image reconstruction for this task is quite compute intensive, therefore it is also advantageously divided in sub-tasks and distributed to different computing sources.

The set of consecutive sub-tasks may comprise a sequential order, if the sub-tasks are all sequentially performed, only by the local, i.e. the "own" medical imaging system unit. However, it is preferably intended to distribute at least one sub-task to a remote computing unit such that some sub-tasks are possibly processed in parallel.

Advantageously, computing resources can be accessed by remote medical imaging systems in a more flexible and optimized manner than in prior art. This is achieved by exchanging the above-mentioned task delegation signals with potentially available remote computing units. Using such a pre-defined and formalized protocol of task delegation signals enables to flexibly combine the computing capacity of any suitable and connectable computing units for performing a computing task in optimized conditions. Hence, a high scalability of the execution of a medical imaging process is achieved, since the number of requestors and executors is only limited by the throughput of the interconnecting infrastructure between the different units, but not any more by the individual processing power of the connected units. Individual remote computing units can vary in processing power or availability but based on their offer during delegation process, they can still compete for a service with a requestor, if their offer is competitive enough. Further, a high flexibility is achieved, since selection rules and an amount of compensation can change on-the-fly in between delegation processes which bring complete flexibility on the choice of an executor. For example, the selection rules can change from processing time to image quality. For example, the requestor might decide to look after the fastest processing algorithm for its tasks immediately after it completed the negotiation and awarded contracts to those executors based on the best image quality criteria. Furthermore, the method according to the invention comprises a high robustness, since executors and requestors can independently decide when to join or to leave the task delegation process.

In the method of performing a sub-task of a remote medical imaging system unit according to the invention, the above-mentioned sequence of signals is exchanged with the remote medical imaging system unit. In other words, the method of performing a sub-task of a remote medical imaging system unit comprises the common and complementary steps to the method of performing a medical imaging process for collectively performing a task using distributed computing units. The method can be performed preferably over a distribution unit. A distribution unit comprises the function of distributing sub-tasks from a medical imaging system unit to an available executor, i.e. an appropriate and available remote computing unit. In this context, it has to be mentioned that the presented method claim is written from the perspective of the remote computing unit and the medical imaging system unit is a remote medical imaging system unit from the perspective of the remote computing unit. The sequence of signals comprises at least a request signal received from the remote medical imaging system unit. The request is related to an execution of a sub-task selected by the remote medical imaging system unit.

The sub-task is received by the remote computing unit which is chosen for performing the sub-task from the remote medical imaging system unit, which is "remote" from the perspective of the remote computing units.

Then, the received sub-task is performed by the chosen remote computing unit and a computing result of the sub-task is generated.

Then, the computing result is transmitted, preferably via the distribution unit, to the remote medical imaging system unit.

Advantageously, the capacity of any idle remote computing units, in particular remote computing units of remote medical imaging systems, is enabled to be used for performing a complex task of a medical imaging system unit and for speeding up the process. This is achieved by exchanging a sequence of task delegation signals with a plurality of remote computing units, preferably via a distribution unit, for selecting at least one of the remote computing units for execution of the selected at least one sub-task. The exchange of the task delegation signals enables to join a current available remote computing unit very flexibly based on deliberately predefined criteria. As later discussed in detail, such criteria may include some parameters of the computing process, for example computing time or a level of image quality achieved by any remote computing unit. In this context, the task delegation process is used for finding out the optimum distribution of a task or the sub-tasks of the task onto at least one, preferably a plurality of remote computing units. Technical parameters also named attributes, achievable by any remote computing units like image quality, required processing time and also the "price to be paid", i.e. the resources, to be spent by the medical imaging system unit for performing these computing processes by the selected remote computing unit or the selected remote computing units, are enabled to be taken into account before a decision of committing a selected remote computing unit has been made. These attributes can also be combined together by weighting them and simple addition or subtraction of these weighted attributes would give a simple value on which a medical imaging system unit can decide about which remote computing unit to choose for performing a selected sub-task.

In the method of distributed performing of a medical imaging process of a medical imaging system according to the invention, a medical imaging system unit of the medical imaging system which is responsible to execute a computing task related to a medical imaging process acts according to the method of performing a medical imaging process. Further, in the above-mentioned method, at least one remote computing unit acts according to the method of performing a sub-task of a remote medical imaging system unit according to the invention, in order to perform at least one sub-task of the medical imaging process. The method of distributed performing of a medical imaging process of a medical imaging system combines the advantages of the method of performing a medical imaging process and of the method of performing a sub-task of a remote medical imaging system unit according to the invention.

The medical imaging system unit according to the invention comprises a division unit for dividing a computing task into a set of sub-tasks. The computing task is related to a generation of medical image data based on measurement data. The division into sub-tasks is performed for distributing parts of the task to different computing resources such that the execution of the task can be accelerated.

The medical imaging system unit also includes a selection unit for selecting a sub-task of the sub-tasks for external execution. The selection unit, for example, selects a sub-task which needs particularly much computing time to solve. By outsourcing such a time-consuming sub-task, the overall computing time can be considerably reduced.

The medical imaging system unit further comprises a communication unit for exchanging a sequence of task delegation signals with a plurality of remote computing units, preferably via a distribution unit for selecting at least one of the remote computing units for execution of the selected sub-task. The sequence of task delegation signals comprises at least one request signal sent by the medical imaging system unit.

Part of the medical imaging system unit is also an outsourcing unit for outsourcing the selected sub-task to the selected remote computing unit for remotely generating a computing result of the selected sub-task. The medical imaging system unit further comprises a reception unit for receiving the generated partial computing result from the selected remote computing unit and also an execution unit for completing the computing task with the computing result received from the selected remote computing unit. The medical imaging system unit shares the advantages of the method of performing a medical imaging process.

The computing unit for performing a sub-task of a remote medical imaging system unit according to the invention comprises a communication unit for exchanging a sequence of signals with the remote medical imaging system unit, preferably over a distribution unit, whereby the sequence of signals comprises at least a request signal received from the remote medical imaging system unit.

The computing unit also comprises a reception unit for receiving the sub-task from the remote medical imaging system unit.

The computing unit further comprises an execution unit for executing the received sub-task and generating a computing result of the sub-task.

The computing unit also includes a transmission unit for transmitting the computing result, preferably via the distribution unit to the remote medical imaging system unit. The computing unit shares the advantages of the method for performing a sub-task of a remote medical imaging system unit.

The medical imaging network according to the invention comprises at least one medical imaging system unit according to the invention, at least one remote computing unit according to the invention and optionally a distribution unit for conveying the communication between the at least one medical imaging system unit and the at least one remote computing unit. The medical imaging network shares the advantages of the method of distributed performing a medical imaging process of a medical imaging system.

A medical imaging network according to the invention may comprise at least one medical imaging system, comprising the medical imaging system unit according to the invention, preferably an MR imaging system. Such a medical imaging system comprises a scan unit for acquiring raw data from an object to be examined and as the medical imaging system unit preferably a local control device. If the medical imaging system comprises an MR imaging system, the local control device may include a measurement control unit and a reconstruction unit for reconstructing image data based on the acquired raw data. A task to be performed by the medical imaging system unit preferably comprises a reconstruction task of the reconstruction unit or a measurement control task of the measurement control unit. Advantageously, these complicated tasks can be completed based on a highly flexible pool of computing resources.

Preferably, the medical imaging system network includes an ensemble of at least two medical imaging systems, wherein each of the at least two medical imaging systems comprises a medical imaging system unit according to the invention and a remote computing unit for remotely generating a partial computing result of the selected sub-task. Advantageously, the different medical imaging systems are enabled to exchange selected sub-tasks dependent on current free calculating capacities such that a medical imaging process of all these medical imaging systems of the ensemble is accelerated as much as possible. In case of a network failure, the system falls back to processing a complete task by one self-contained medical imaging system.

The remote computing unit preferably comprises a medical imaging system unit itself or alternatively a so called compute only source. A compute-only source is not necessarily assigned to a complete medical imaging system, quite the contrary, it may also include only a computer for calculating results of sub-tasks. Advantageously, the at least one compute-only source enables to further speed up the medical imaging process. For example, the medical imaging system network preferably includes a dedicated computing cluster or one or more highly-performant computing machines with a single task of offering free computing resources that may be used by a medical imaging system which is still self-contained and can operate the complete task on its own in case of network issues. A big advantage of the medical imaging system network is that the additional computing power, i.e. the additional compute-only source, can easily be extended or renewed with a significantly higher frequency than the medical imaging system itself to benefit from the very short innovation cycles in the computing industry. Such compute-only source can also be provided by a cloud computing system with of course higher latencies but for some use cases this may also be beneficial, for example, if a very high amount of computation work is required only in rare cases.

Some units or modules of the medical imaging network mentioned above can be completely or partially realized as software modules running on a processor of a respective computing system unit, e.g. on a local control device of a medical imaging system, in particular a magnetic resonance imaging system or a CT-system, or on a remote computing unit.

A realization largely in the form of software modules can have the advantage that applications already installed on an existing computing system can be updated, with relatively little effort, to install and run these units of the present application. The object of the invention is also achieved by a computer program product with a computer program that is directly loadable into the memory of a computing system, and which comprises program units to perform the steps of the inventive methods, at least those steps that could be executed by a computer, when the program is executed by the computing system. In addition to the computer program, such a computer program product can also comprise further parts such as documentation and/or additional components, also hardware components such as a hardware key (dongle etc.) to facilitate access to the software.

A computer readable medium such as a memory stick, a hard-disk or other transportable or permanently-installed carrier can serve to transport and/or to store the executable parts of the computer program product so that these can be read from a processor unit of a computing system. A processor unit can comprise one or more microprocessors or their equivalents.

In that context, it has to be mentioned that negotiation scenario description and runtime environment constitute contextual and conversation design limits for the negotiation and bidding process of multi-unit systems. The orientation to language-independent description of a negotiation scenario as well as ready-to-use platform for provision of remote computing efficiency logic without hurdle of implementing specific communication layers define a major criterion in choice of such software platform. Preferably, SDLMAS language and platform can be chosen for realizing the software modules for the medical imaging network. Such a concept is described in Cavrak, Igor, Armin Stranjak, and Mario Zagar (2009) "SDLMAS: A Scenario Modelling Framework for Multi-Agent Systems" Journal of the JUCS 15(4): 898-925 and Stranjak, Armin, Igor Cavrak, and Mario Zagar (2008) "Scenario Description Language for Multi-Agent Systems" In: Nguyen, N.T., Borzemski, L., Grzech, A., Ali, M. (Eds.) IEA/AIE LNCS (LNAI) (Springer, Heidelberg) vol. 5027: 855-864. SDLMAS explicitly provides expressive language for scenario description, independence between implementation details of an efficiency logic of different units and provision of a platform including code generation of necessary communication stack. It was also proved to be a convenient platform for application in task scheduling. This is described in Stranjak, Armin, Igor Cavrak and Mario Zagar (2011) "Scenario-Description Language in Multi-Agent Simulation System" in O'Shea, J. et al. (Eds.): KES-AMSTA LNAI (Springer-Verlag Berlin Heidelberg) 6682: 169-179.

The dependent claims and the following description each contain particularly advantageous embodiments and developments of the invention. In particular, the claims of one claim category can also be further developed analogously to the dependent claims of another claim category. In addition, within the scope of the invention, the various features of different exemplary embodiments and claims can also be combined to form new exemplary embodiments.

In a variant of the method of performing a medical imaging process according to the invention, the step of exchanging the sequence of signals includes the sub-step of sending a first request signal, also named a "call request signal", to the plurality of remote computing units, preferably via the distribution unit. The first request signal is intended to request as much remote computing units as possible to send their service proposals to the requesting medical imaging system unit. Further, the step of exchanging the sequence of signals comprises the sub-step of receiving a service proposal signal from at least one remote computing unit, preferably a group of one or preferably more remote computing units of the plurality of remote computing units, preferably via the distribution unit. Furthermore, the step of exchanging the sequence of signals comprises the sub-step of selecting at least one remote computing unit, preferably a group of the plurality of remote computing units, for executing the selected sub-task based on the received service proposal signal. A further sub-step comprises the step of sending a second request signal, preferably an outsourcing request signal for execution of the selected sub-task, to the selected remote computing unit and the step of receiving a commitment signal of the at least one remote computing unit for execution of the selected sub-task or a withdrawal of the proposal.

Preferably, the step of sending a second request signal comprises transmitting an offer to the selected, at least one currently freely available remote computing unit containing details of a criteria for a compensation of a remote execution of the selected sub-task with a quantitatively defined access to resources. Preferably, the offer contains the details about the functional definition of the award function including already given values of the award function.

Preferably, the step of receiving a commitment signal comprises receiving a rejection or acceptance from the selected remote computing unit.

Preferably, the step of exchanging the coupling sequence of signals comprising task delegation signals includes at least one of the steps: a step of transmitting an answer signal to the selected remote computing unit. The answer signal includes an information of decline or commit for the compensation of a remote execution of the selected sub-task with a quantitatively defined access of the remote execution to resources, preferably technical resources. The quantitatively defined access to resources comprises a resource balancing that may comprise a "payment" based on an agreed award function. The "payment" may comprise the right to use technical resources of the requesting medical imaging system unit, it also may include something like a real payment using monetary compensation. The right to use technical resources preferably comprises exclusive rights for computing time or a priority right to access to computing power of the requesting medical imaging system unit.

The step of exchanging the coupling sequence of task selection signals preferably comprises the step of receiving an answer signal (or "re-answer signal") to the answer signal from the selected remote computing unit including an information message including the preferably partial computing result of the selected sub-task or no result. Advantageously, the requesting medical imaging system unit receives in each case an information about the completion of the computing process of the remote computing unit. In case a partial result is received, the medical imaging system unit is enabled to complete its task based on the partial result. In case the medical imaging system unit receives an information about the fact that no result is generated by the remote computing unit until a predetermined point of time, the medical imaging system unit is enabled to react to such a dysfunction by redistributing the sub-task or by completing the selected sub-task by itself.

The step of exchanging the coupling sequence of signals also preferably comprises the step of transmitting a compensation signal. The compensation signal comprises an information about a compensation of the remote execution of the selected sub-task by a quantitatively defined access to resources to the selected remote computing unit. Such a compensation preferably comprises an award for the execution of the selected sub-task. The amount of the compensation, preferably an award, is preferably based on the reception time of the result and the offer. Hence, the compensation is scalable and adaptable to the performance of the selected remote computing unit such that there is a stimulus for the remote computing unit to perform the selected sub-task with the highest possible performance.

In a variant of the method of performing a medical imaging process according to the invention, the request signal, which may be the first and/or the second request signal, comprises details about the sub-task to be executed. These details preferably comprise an algorithm name and version which are known to the currently freely available remote computing unit in advance and/or criteria for a compensation of a remote execution of the selected sub-task with a quantitatively defined access to resources, in particular the award promised to the winning bidder.

Preferably, the service proposal signal comprises an estimated time, by when the sub-task can be executed by the remote computing unit. Advantageously, the medical imaging system unit is enabled to select a remote computing unit for performing the selected sub-task based on an estimated time for the execution of the sub-task. In particular, the medical imaging system unit is enabled to select a remote computing unit, the estimated time of which matches to the time planned by the medical imaging system unit for the sub-task.

In a variant of the method of performing a medical imaging process according to the invention, the compensation signal, transmitted to the selected remote computing unit, comprises an award for compensation of execution of the selected sub-task. Advantageously, the award is used as a means for compensation of the remote execution of the selected sub-task.

Preferably, the request signal, which may be the first and/or the second request signal, comprises a preferably guaranteed award value if the sub-task is executed at the estimated time estimated by the selected remote computing unit, and a maximum award value if the sub-task is executed earlier. Advantageously, there is a motivation for the selected remote computing unit to perform the selected sub-task as fast as possible.

In a variant of the method of performing a medical imaging process according to the invention, the step of sending a second request signal comprises transmitting an offer from the requesting medical imaging system unit to the selected remote computing unit and the offer includes a criteria for a compensation of a remote execution of the selected sub-task with a quantitatively defined access to resources and the criteria includes details about the shape of the functional definition of an award function. The award function preferably includes an already given guaranteed award value, a maximum award value as well as a time of deadline, representing the deadline after which an award value will not be offered. Advantageously, the selected remote computing unit is informed about the details of the offered compensation, such that it is enabled to make a realistic offer of performing the selected sub-task. The deadline enables to calculate a time limit, until the selected sub-task will be completed by the selected remote computing unit. Otherwise, the remote control unit does not get any compensation for executing the sub-task.

Preferably, the task to be performed comprises a reconstruction task for parallel image reconstruction based on sub-sets of parallel acquired raw data and each of the sub-tasks comprises a reconstruction of a partial image based on a different sub-set. Advantageously, these sub-tasks are enabled to compute in parallel using the methods according to the invention.

Preferably, the computing task includes at least one of the following types of control tasks: controlling a scan unit of the medical imaging system, or reconstructing image data based on raw data acquired by a scan unit of the medical imaging system. Advantageously, the time requirement for completing these tasks can be reduced by performing sub-tasks in parallel by selected remote computing units.

Preferably, the execution unit of the medical imaging system unit is arranged to locally execute a sub-task, if an outsourcing of this sub-task is not appropriate according to a predefined criterion. A predefined criterion may comprise that the local execution is more efficient, preferably with respect to aspects of time consumption or existence of free local computing resources.

In other words, the outsourced sub-task is locally executed by the execution unit of the local medical imaging system unit by default. "By default" means that the outsourced sub-task is executed by the local execution unit based on the above-mentioned criterion. The above-mentioned criterion preferably includes the decision of a locally execution of the sub-task in case no result is received from the remote computing unit processing the outsourced sub-task. Advantageously, the local control unit is self-contained.

In a variant of the medical imaging system unit according to the invention, the execution unit is arranged to detect, as a special type of predefined criterion, the status of one or more completed consecutive sub-tasks to determine based on the detected status if the computing result generated by the remote computing unit is received by the medical imaging system unit or will be received on time. Hence, it is detected if it is necessary to start the related sub-task by the medical imaging system unit by default. Further, the medical imaging system unit is arranged to execute the computing task based on the received computing result if the partial computing result is received on time, or alternatively to execute the selected sub-task itself if the partial computing result is not received on time. Advantageously, the computing task can be completed in any case without any delay compared to a completely local execution of the computing task.

In a variant of the medical imaging system unit according to the invention, a task to be computed includes at least one of the following types of tasks:
- controlling a scan unit of the related medical imaging system,
- reconstructing image data based on raw data acquired by a scan unit of the medical imaging system.

Advantageously, the generation of control data or a sub-task of a reconstruction task can be relocated to different computing resources for accelerating a control process or reconstruction process.

In another variant of the medical imaging system unit according to the invention, the sequence of tasks to be processed is implemented as a queue of consecutive sub-tasks. The queue determines an unambiguous order of processing the consecutive sub-tasks, particularly in case the sub-tasks are performed by the medical imaging system unit. Advantageously, an order for processing the different sub-tasks is generated. For example, the order can be determined based on available input data or based on the consumption of time the different sub-tasks need.

In a further variant of the medical imaging system unit according to the invention, the remote computing unit includes a remote medical imaging system unit of a different medical imaging system. Advantageously, computing resources of medical imaging systems, which are usually only used when an imaging process is running, can be exploited over the whole time, if they are accessed by remote medical imaging systems.

Preferably, in a variant of the medical imaging system unit according to the invention, the remote computing unit includes a cluster comprising a plurality of medical imaging system units of different medical imaging systems. Advantageously, every medical imaging system that has an idle computing power can be used to execute sub-tasks, thus they form a kind of virtual cluster, distributed and dynamically available. Further, the local medical imaging system unit according to the invention itself may be arranged to offer its computing power to other remote medical imaging systems if it is idle itself.

In a preferred variant of the medical imaging system unit according to the invention, the remote computing unit is located in a cloud computing system. Advantageously, the remote computing source can also be provided as virtual resource in a cloud computing system without an actual existence of an additional remote medical imaging system.

Preferably, the medical imaging system according to the invention comprises at least one of:
- a magnetic resonance imaging system,
- an X-ray imaging system, preferably a CT-imaging system,
- an ultrasound imaging system.

A magnetic resonance imaging method comprises, additionally to the acquisition of raw data, a plurality of processing steps. These processing steps preferably include processing steps for preparing the raw data for reconstruction of image data, the reconstruction of image data itself and further steps for generating image data, for example the application of additional reconstruction algorithms, the combination of partial images to a combined image and post-processing steps for corrections of movements. Further, the steps for generating image data preferably comprise at least one of: steps for generating an evaluation of time series, steps for detection of anatomical structures, steps for performing a functional magnetic resonance imaging, steps for performing a diffusion imaging, steps for performing a perfusion imaging and steps for performing filtering methods.

Preferably, the reconstruction is divided into a plurality of sub-tasks, which are transmitted to remote computing resources. Further, also the following steps like application of additional reconstruction algorithms, post-processing steps for corrections of movements, evaluation of time series, detection of anatomical structures, functional magnetic resonance imaging, diffusion imaging, perfusion imaging and filtering methods, are preferably divided into sub-tasks and outsourced to remote computing units.

Input data for a sub-task are typically either pre-processed raw-data or reconstructed images (the latter especially for post-processing), but this is strongly dependent on the concrete algorithm or even variant. Intermediate points could make sense, but in practice, these two are the most relevant ones. For example, both filtering or motion correction can be done either on early stages in the image recon pipeline or on the reconstructed images, depending on the concrete algorithm. In practice, we have to identify a concrete point in the reconstruction pipeline at which it makes sense for the individual concrete use case from which on the external computation would make sense. For example, in case of alternative reconstructions on the basis of the same raw data this would be at a very early stage of the pipeline (directly after system-dependent pre-processing/normalization of the raw data), even if the original reconstruction offloaded only e.g. post-processing tasks (e.g. because the medical images, in particular MR images, are acquired sequentially over time).

A CT-imaging method comprises the steps of receiving raw data from a scan unit, a preparation step for generating corrected and re-arranged raw data. The actual CT-reconstruction step for generating image data based on the prepared raw data and a filtering step. In CT-imaging, a plurality of different sets of image data can be generated based on the same set of raw data using different sets of parameters for reconstruction. All these reconstructions can be implemented as sub-tasks performed on different remote computing units. Further, a filtering step is carried out on the generated image data. Also the filtering step can be carried out by the different remote computing units or alternatively by a local medical imaging system unit.

The invention is explained below with reference to the figures enclosed once again. The same components are provided with identical reference numbers in the various figures. The figures are usually not to scale.
FIG 1 shows a flow chart illustrating a method of performing a medical imaging process of an MR imaging system according to an embodiment of the invention,
FIG 2 shows a flow chart illustrating step 1.III of the method of performing a medical imaging process of an MR imaging system shown in FIG 1,
FIG 3 shows a flow chart illustrating sub-steps 1.IIIf to 1.IIIh of step 1.III of the method of performing a medical imaging process of an MR imaging system shown in FIG 1,
FIG 4 shows a flow chart illustrating a method for executing a sub-task of a medical imaging system unit according to an embodiment of the invention,
FIG 5 shows a flow chart illustrating step 4.1 of the method of performing a sub-task of a medical imaging system unit shown in FIG 4,
FIG 6 shows a schematic diagram illustrating the communication in a medical imaging network according to an embodiment of the invention,
FIG 7 shows a schematic representation illustrating a medical imaging network according to an embodiment of the invention,
FIG 8 shows a schematic diagram illustrating the communication in a medical imaging network according to a second embodiment of the invention,
FIG 9 shows a schematic representation illustrating a medical imaging network according to a second embodiment of the invention,
FIG 10 shows a graphical representation of an award function depending on the time,
FIG 11 shows a schematic view of a medical imaging system unit according to an embodiment of the invention,
FIG 12 shows a schematic view on a computing unit for performing a task of a medical imaging system unit according to an embodiment of the invention.

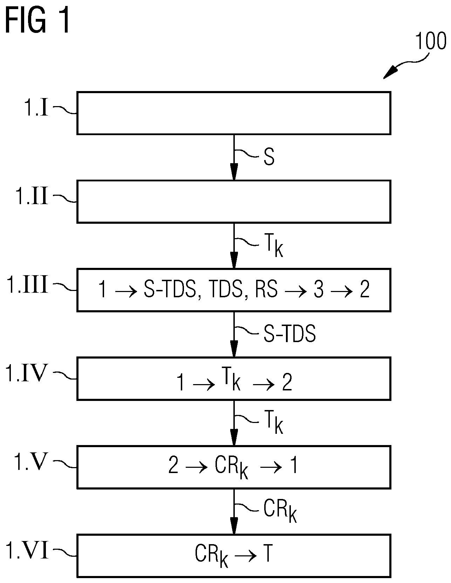

FIG 1 shows a flow chart 100 illustrating a method of performing a medical imaging process of an MR imaging system according to an embodiment of the invention.

In step 1.1, a computing task T is received by a medical imaging system unit 1, for example from a control unit of a medical imaging system, and the received task T is divided into a set S of sub-tasks T_{ι}, ..., Tₙ. The task T to be processed is related to a generation of medical image data based on measurement data.

In step 1. II, one sub-task Tₖ of the sub-tasks T_{ι}, ..., Tₙ of the set S of sub-tasks T_{ι}, ..., Tₙ is selected for external execution (n is a natural number, 1 <= k <= n).

In step 1.III, a sequence S-TDS of task delegation signals TDS is exchanged with a plurality of remote computing units 2 via a distribution unit 3, for selecting at least one of the remote computing units 2 for execution of the selected sub-task Tₖ. The sequence S-TDS of task delegation signals TDS comprises at least one request signal RS sent by the medical imaging system unit 1.

In step 1. IV, the selected sub-task Tₖ is outsourced to the selected remote computing unit 2 for remotely generating a computing result CRₖ of the selected sub-task Tₖ.

In step 1.V, the generated computing result CRₖ is received by the medical imaging system unit 1 from the selected remote computing unit 2.

In step l.VI, the computing task T is completed based on the computing result CRₖ received from the selected remote computing unit 2.

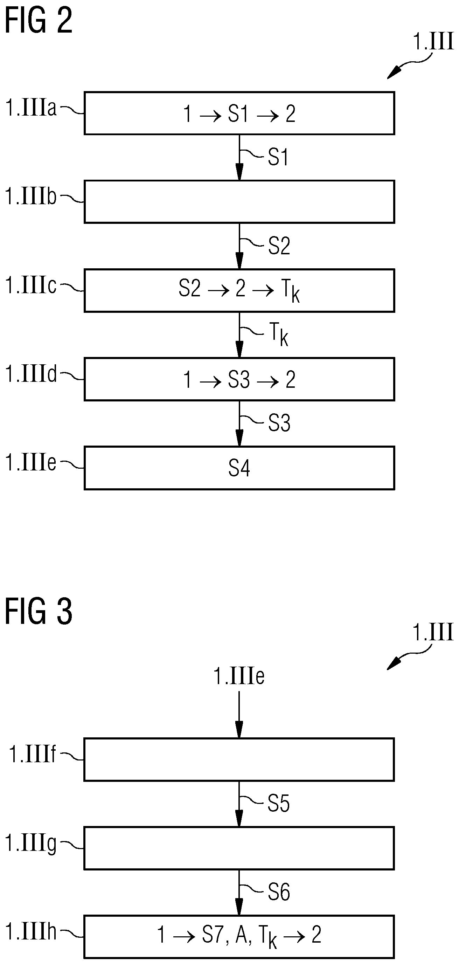

In FIG 2, a flow chart concerning step 1.III of the method of performing a medical imaging process of an MR imaging system shown in FIG 1 is illustrated.

In step 1.III, exchanging of a sequence S-TDS of signals includes the following sub-steps:
In sub-step l.IIIa, a first request signal S1, i.e. a "call request signal", is sent as a kind of broadcast signal via the distribution unit 3 to the plurality of remote computing units 2. The "call request signal" requests all available remote computing units 2 for sending a service proposal signal S2 to the requesting medical imaging system unit 1.

In sub-step l.IIIb, a service proposal signal S2 is received from a group of the remote computing units 2 which have received the first request signal S1 and are in principle available for performing a sub-task Tₖ of the requesting medical imaging system unit 1.

In sub-step l.IIIc, at least one remote computing unit 2 of the group of remote computing units 2 is selected by the medical imaging system unit 1 for executing the selected sub-task Tₖ based on the received service proposal signal S2.

In sub-step 1.IIId, a second request signal S3, i.e. an outsourcing request signal is sent for execution of the selected sub-task Tₖ to the selected remote computing unit 2.

In sub-step l.IIIe, a commitment or proposal withdrawal signal S4 of the selected remote computing unit 2 for execution of the selected sub-task Tₖ is sent from the selected remote computing unit 2 to the medical imaging system unit 1.

FIG 3 shows a flow chart illustrating optional sub-steps 1.IIIf to 1.IIIh of step 1.III of the method of performing a medical imaging process of an MR imaging system shown in FIG 1.

In sub-step l.IIIf, an answer signal S5 is transmitted to the selected remote computing unit 2 including an information of decline or commit for the compensation of a remote execution of the selected sub-task Tₖ with a quantitatively defined access to resources. Such a quantitatively defined access to resources can be realized as a payment based on an agreed award function. In that context, it has to be mentioned that a "payment" is not restricted to a commercial transaction, it may also include a formal clearing of supplied performances.

In sub-step l.IIIg, an answer signal S6 to the answer signal S5 to the selected remote computing unit 2 is received by the medical imaging system unit 1 from the selected remote computing unit 2. The answer includes an information message including the partial computing result CRₖ of the selected sub-task Tₖ or no result for the case that the remote computing unit 2 was not able to perform the sub-task Tₖ and to provide the expected partial computing result CRₖ.

In sub-step l.IIIh, a compensation signal S7, comprising an award A as a compensation of the remote execution of the selected sub-task Tₖ is transmitted from the medical imaging system unit 1 to the selected remote computing unit 2. The award A is a type of a quantitatively defined access to resources. The award A can be determined based on the actual reception time Tᵣ of the result CRₖ and the offer.

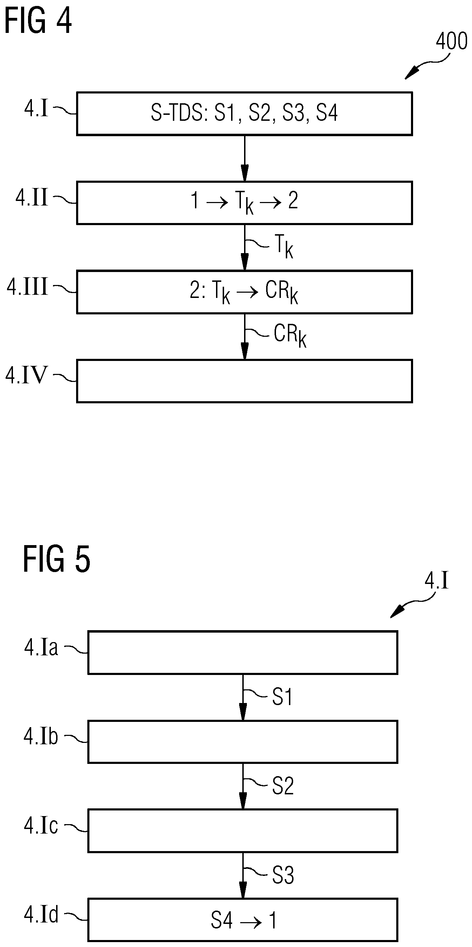

FIG 4 shows a flow chart 400 illustrating a method for performing a sub-task Tₖ of a remote medical imaging system unit 1 according to an embodiment of the invention. The method is described in the perspective of the performing remote computing unit 2.

In step 4.1, a sequence S-TDS of task delegation signals TDS is exchanged by the remote computing unit 2 with the remote medical imaging system unit 1 over a distribution unit 3, whereby the sequence S-TDS of signals TDS comprises a request signal S1 for execution of the sub-task Tₖ received from the remote medical imaging system unit 1.

In step 4.11, the sub-task Tₖ is received from the remote medical imaging system unit 1.

In step 4.111, the received sub-task Tₖ is executed and a computing result CRₖ of the sub-task Tₖ is generated by the remote computing unit 2.

In step 4.IV, the computing result CRₖ is transmitted via the distribution unit 3 to the remote medical imaging system unit 1.

In FIG 5, a flow chart illustrating step 4.1 of the method for executing a sub-task Tₖ of a medical imaging system unit 1 shown in FIG 4 is depicted. Step 4.1 comprises the step of exchanging a sequence S-TDS of task delegation signals TDS.

In sub-step 4.Ia, a first request signal S1, i.e. a call request signal, is received by the remote computing unit 2 from the medical imaging system unit 1 via the distribution unit 3.

In sub-step 4.Ib, a service proposal signal S2 is sent from the remote computing unit via the distribution unit 3 to the medical imaging system unit 1.

In sub-step 4.Ic, a second request signal S3, i.e. an outsourcing request signal for execution of the selected sub-task Tₖ is received by the selected remote control unit 2 from the medical imaging system unit 1.

In sub-step 4.Id, a commitment or proposal withdrawal signal S4 for execution of the selected sub-task Tₖ is sent from the selected remote control unit 2 to the medical imaging system unit 1.

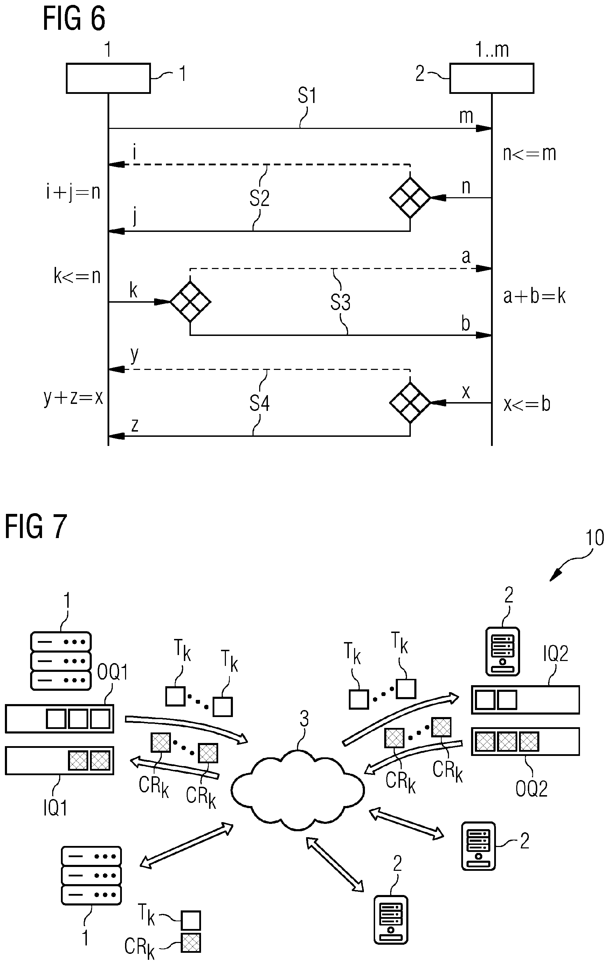

In FIG 6, a schematic diagram illustrating the communication in a medical imaging network 10 (shown in FIG 7) according to an embodiment of the invention is shown.

On the upper left side of the schematic diagram, a medical imaging system unit 1, also named as requestor, is depicted and on the right side of the schematic diagram, a remote computing unit 2, also named as executor, is depicted. The medical imaging system unit 1 on the left side symbolizes one single medical imaging system unit 1 (symbolized by the number "1" above the schematic rectangle related to the medical imaging system unit 1. However, the remote computing unit 2 on the right side symbolizes a plurality of m remote computing units 2 (m is a natural number), which is illustrated by "1..m" above the schematic rectangle symbolizing the remote computing units 2.

In a first step, a service request signal, i.e. a "call for service" S1, is sent to all m registered remote computing units 2 of the medical imaging network 10. In a second step, the medical imaging system unit 1 waits for an answer from the remote computing unit 2 with a predetermined deadline.

Further, the medical imaging system unit 1 receives some service proposal or refusal signals S2 from n remote computing units 2, wherein n is a natural number and n <= m. Indeed, only j remote computing units 2 send a service proposal to the medical imaging system unit 1 and i remote computing units 2 send a service refusal back to the requesting medical imaging system unit 1, wherein i, j are natural numbers and i + j = n. The square standing on a corner symbolizes the possible different types of answer, i.e. the service proposal or refusal.

The medical imaging system unit 1 considers the proposals from the j remote computing units 2 based on an internal logic. The internal logic can be based on a resource allocation system, wherein provided resources are quantitatively cleared. For example, such a resource includes an amount of provided processing time and the remote computing unit 2 with the shortest processing time is favoured.

In a third step, based on an internal selection, the medical imaging system unit 1 transmits a signal S3 to k remote computing units 2 (k is a natural number and k <= n) including information about an acceptance or rejection of their bids. For example to a number of "a" remote computing units 2 (a is a natural number) a rejection is sent and to a number of b remote computing units 2 (b is a natural number) a proposal of acceptance is sent, wherein a + b <= k.

In a fourth step, a commitment or proposal withdrawal signal S4 is sent from the remote computing units 2 to the medical imaging system unit 1. In detail, x remote computing units 2 (x is a natural number and x <= b) send a final answer to the medical imaging system unit 1. A number of z remote computing units 2 (z is a natural number and z <= x) send a final commitment message to the medical imaging system unit 1 and this message will bind both sides into a contracting obligation. Further, y remote computing units 2 send a proposal withdrawal to the medical imaging system unit 1 (y is a natural number and y + z = x) .

In FIG 7, a schematic representation illustrating a medical imaging network 10 according to an embodiment of the invention is depicted.

The medical imaging network 10 comprises a plurality of medical imaging system units 1, a plurality of remote computing units 2 and a distribution unit 3. The distribution unit 3 provides a type of technical distribution node for connecting and assigning requesting medical imaging system units 1 and potential executing remote computing units 2. Each of the medical imaging system units 1 includes an output queue OQ1 including sub-tasks Tₖ to be outsourced to the remote computing units 2 and an input queue IQ1 for receiving results CRₖ of executed sub-tasks Tₖ. Correspondingly, each remote computing unit 2 also comprises an input queue IQ2 for receiving sub-tasks Tₖ to be executed and an output queue OQ2 for outputting results CRₖ to the remote medical imaging system units 1 from which the corresponding sub-tasks Tₖ have been received. The distribution unit 3 receives and distributes these transmitted sub-tasks Tₖ and results CRₖ to the addressed units 1, 2.

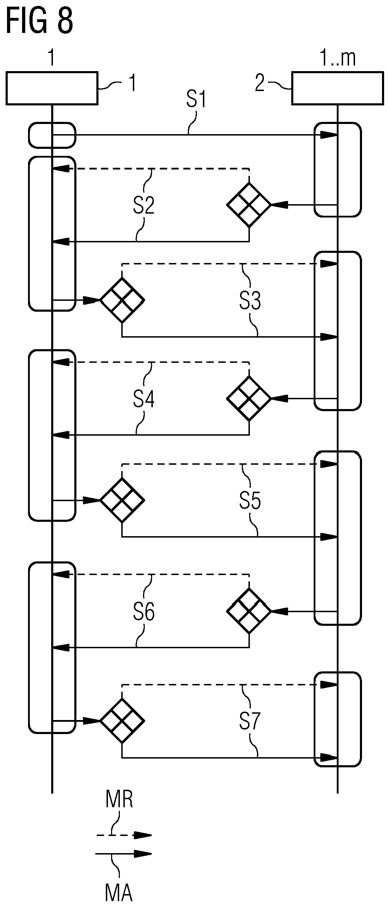

In FIG 8, a schematic diagram illustrating the communication in a medical imaging network according to a second embodiment of the invention is depicted. Messages of rejection MR are symbolized by an arrow with a dashed line and messages of acceptance MA include an arrow with a solid line.

Similarly to the embodiment shown in FIG 6, on the upper left side of the schematic diagram, a medical imaging system unit 1, also named as requestor, is depicted and on the right side of the schematic diagram, a remote computing unit 2, used as executor, is depicted. The medical imaging system unit 1 on the left side symbolizes one single medical imaging system unit 1 (symbolized by the number "1" above the schematic rectangle related to the medical imaging system unit 1). However, the number of one medical imaging system unit 1 is only chosen for the sake of simplicity and it is explicitly mentioned that the invention is also directed to a plurality of medical imaging system units 1. Similar to FIG 6, the remote computing unit 2 symbolizes a plurality of m remote computing units 2 (m is a natural number).

In a first step, as soon as a requestor identifies a need for additional resources to complete its task T, the requestor 1 starts negotiation conversation with all identified and available executors 2.

This is formally initiated with the transmission of a message named a "call for proposal" (by short CFP) S1, which is sent to all m registered remote computing units 2. The content of this "call for proposal" message contains the details about the task that needs to be executed. These details typically include an algorithm name and a version number, which are known to the executors 2 in advance, and the award A promised to the winning bidder 2. The data values needed for task execution, normally do not have any significant influence on the execution time and therefore the data are provided later only to the winning executor 2.

In a second step, the medical imaging system unit 1 waits for an answer from the remote computing units 2 with a predetermined deadline. All these potential executors 2 consider a task execution request and provide their bids.

Based on an award function, the requestor 1 provides only a guaranteed award value A₀ if the sub-task Tₖ is executed at the time estimated by the winning executor 2 as well as a maximum award value Aₘ if the task is executed earlier. Based on these two values A₀, Aₘ, executors 2 consider such offers and by sending a message with a performative PROPOSE (solid line), they provide the estimated time Tₑ which runs out, when the sub-task Tₖ is completed. Alternatively, the executors 1 simply reject the "call for proposal" by sending terminating performative NO-PROPOSAL (dashed line). Upon reception of all bids or the expiration of a given timeout period, the requestor 1 chooses the winning bid from the received vector of estimated times Tₑ by selecting the executor 2 with the shortest estimated time Tₑ for the sub-task execution. In order to ensure that executors 2 will use their dominant bidding strategy by truthfully revealing their time execution evaluation, the negotiation is based on second best estimation auction (Vickrey auction). Therefore, the requestor 1 chooses the winning executor 2, but it offers estimated time Tₑ from the second best offer. In such way, it is in the executor's interest to bid truthfully their estimation.

Hence, in the second step, the medical imaging system unit 1 receives some service proposal or refusal signals S2 from n remote computing units 2, wherein n is a natural number and n <= m.

The medical imaging system unit 1 considers proposals from the remote computing units 2 based on an internal logic. The internal logic can be based on a resource allocation system, wherein provided resources are quantitatively cleared. For example, the resources include an amount of provided processing time and the shortest processing time is favoured.

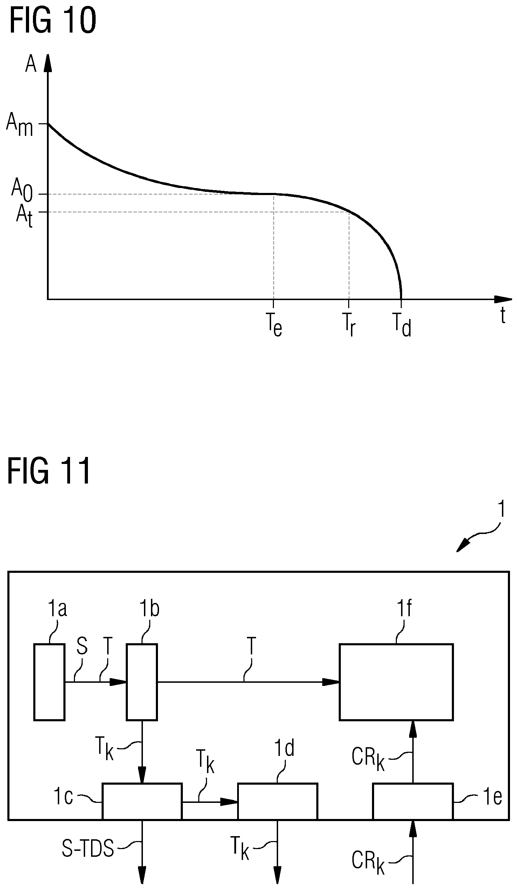

In a third step, based on an internal selection, the medical imaging system unit 1 transmits a signal S3 to k (k is a natural number and k <= n) of the remote computing units 2 including information about an acceptance or rejection of their bids. For example to a number of "a" remote computing units 2 (a is a natural number) a rejection is sent and to a number of b remote computing units 2 (b is a natural number) a proposal acceptance is sent, wherein a + b <= k. In the third step, the winning executor 2 now receives a signal S3 including an offer message that contains the details about the functional definition of the award function A, including already given values of the guaranteed award value A₀ and the maximum award value Aₘ as well as the time T_{d} representing the deadline after which an award value will not be offered. The shape of such function would depend on the mathematical definition. In FIG 10, an illustrative example of such an award function A is depicted.

In a fourth step, using a signal S4, x remote computing units 2 (x is a natural number and x <= b) send a final commitment message or a proposal withdrawal to the medical imaging system unit 1 and, in case it includes a final commitment message, this message will bind both sides into a contracting obligation.

The executor 2 considers the received offer reflected in key values of an award function A and it bids truthfully its estimation of time Tₑ needed to execute the given sub-task Tₖ by replying an ACCEPT message. Should it choose to reject the offer, it will send a REJECT message.

In a fifth step, an answer signal S5 is transmitted to the selected remote computing unit 2 including an information of decline or commit for the compensation of a remote execution of the selected sub-task Tₖ with a quantitatively defined access to resources. If a requestor 1 intends to proceed with the agreement, it replies with a COMMIT message (arrow with solid line) formally accepting the bid and committing itself for the payment based on agreed award function A. Alternatively, it will reply with a decline message (arrow with dashed line).

As soon as the sub-task Tₖ is processed, the result will be provided by the executor 2, i.e. the remote computing unit 2, by sending a signal S6, including an INFORM message (arrow with solid line) to the requestor, i.e. the medical imaging system unit 1, and if the task execution is given up, a NO-RESULT message (arrow with dashed line) is transmitted to the requestor 1. In other words, in the sixth step, an answer signal S6 to the answer signal S5 to the selected remote computing unit 2 is received from the selected remote computing unit 2 including an information message comprising the computing result CRₖ of the selected sub-task Tₖ (arrow with solid line) or no result (arrow with dashed line). Time elapsed between transmission of the COMMIT message (signal S5) and reception of the INFORM message (signal S6) is used as the time of result reception Tᵣ. This value is crucial to calculate actual award Aₜ that will be transferred from requestor 1 to executor 2 upon the reception of the task execution result CRₖ. In case shown in FIG 10, time Tᵣ is longer than estimated time Tₑ and therefore that award transferred is lower than agreed value of A₀. Should executor 2 perform the sub-task Tₖ sooner estimated Tₑ, it would receive a value higher than A₀, per agreed award function A. As mentioned earlier, award function shape is agreed during negotiation.

In the seventh step, a compensation signal S7, comprising a compensation of the remote execution of the selected sub-task Tₖ by a quantitatively defined access to resources is sent to the selected remote computing unit 2. That quantitatively defined access to resources includes the above-mentioned award. If the executor 2 which performed the sub-task Tₖ is from an internal network system, the award transferred has only an intrinsic value within that network system. In this case, the received award can be used in the future when that executor 2 will undertake a role of a requestor. Such award can be generated and distributed locally to the executors 2 in form of encrypted award tokens or some sort of cryptocurrency. Accumulation of award values would help the executor to offload more tasks and therefore help in fulfilling the interests of executing its own tasks promptly and optimally in the future. On the other hand, payment to a cloud executor may include an award with actual financial value per contractual obligation with the cloud executor.

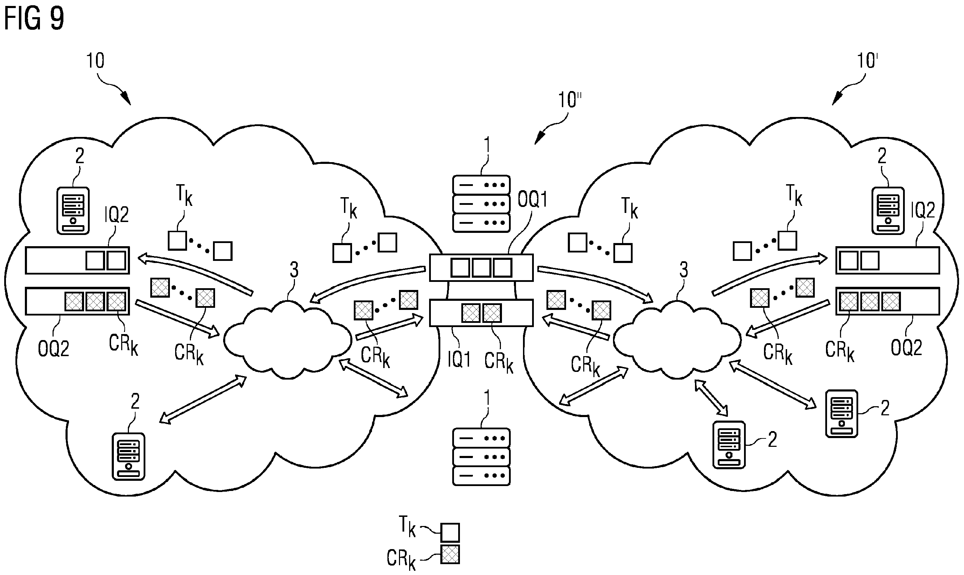

In FIG 9, a schematic representation illustrating a medical imaging network 10'' according to a second embodiment of the invention is depicted. In contrast to the medical imaging network 10 according to the first embodiment shown in FIG 7, the medical imaging network 10'' includes a local part 10 shown on the left side of FIG 9 and a global part 10', for example implemented by a cloud computing system, shown on the right side of FIG 9. The local part 10 and the global part 10' are substantially illustrated in a mirrored way, since the medical imaging system unit 1 is illustrated in the centre of FIG 9.

The local part 10 and the global part 10' comprise a plurality of remote computing units 2 and a distribution unit 3. The distribution unit 3 provides a type of technical distribution node for connecting and assigning requesting medical imaging system units 1 (shown in the centre between the local part 10 and the global part 10') and potential executing remote computing units 2. The medical imaging system units 1 include an output queue OQ1 including sub-tasks Tₖ to be outsourced to the remote computing units 2 and an input queue IQ1 for receiving results CRₖ of executed sub-tasks Tₖ. Correspondingly, each remote computing unit 2 also comprises an input queue IQ2 for receiving sub-tasks Tₖ to be executed and an output queue OQ2 for outputting results CRₖ to the remote medical imaging system units 1 from which the corresponding sub-tasks Tₖ have been received. The distribution unit 3 receives and distributes these transmitted sub-tasks Tₖ and results CRₖ to the addressed units 1, 2. By integrating the cloud computing system 10' into the medical imaging network 10", a high flexibility and scalability of the medical imaging network 10'' is achieved.

FIG 10 shows a graphical representation of an award function A depending on the time t. The shape of the award function A can be arbitrarily chosen by a requestor 1. It typically reflects an urgency and importance of a sub-task Tₖ, e.g. by providing higher A₀ and Aₘ values and/or a steeper functional curve before Tₑ, or a time-sensitivity of the sub-task Tₖ, for example by providing a hard deadline and no award after Tₑ, where T_{d} = Tₑ. A₀ represents the guaranteed award value and Aₘ represents the maximum award value. Tₑ represents the estimated time for execution of a sub-task Tₖ, Tᵣ is the actual time of result reception or reception time and T_{d} represents the deadline after which an award value will not be offered. The value Aₜ represents the value actually achieved based on the time Tᵣ of result reception. Hence, the "guaranteed award value" A₀ is only achieved if the estimated Time Tₑ is retained.

FIG 11 shows a schematic view on a medical imaging system 1 unit according to an embodiment of the invention. The medical imaging system unit 1 comprises a division unit 1a for dividing a computing task T into a set S of sub-tasks T_{ι}, ..., Tₙ.

The medical imaging system unit 1 also comprises a selection unit 1b for selecting a sub-task Tₖ of the sub-tasks T_{ι}, ..., Tₙ for external execution.

Part of the medical imaging system unit 1 is also a communication unit 1c for exchanging a sequence S-TDS of task delegation signals TDS with a plurality of remote computing units 2 (not shown in FIG 11), preferably via a distribution unit 3 (not shown in FIG 11), for selecting at least one of the remote computing units 2 for execution of the selected sub-task Tₖ.

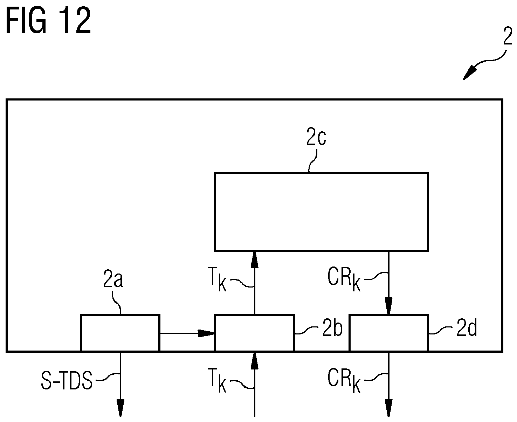

The medical imaging system unit 1 also comprises an outsourcing unit 1d for outsourcing the selected sub-task Tₖ to the selected remote computing unit 2 (shown in FIG 12) for remotely generating a computing result CRₖ of the selected sub-task Tₖ.

The medical imaging system unit 1 also includes a reception unit le for receiving the generated partial computing result CRₖ from the selected remote computing unit 2.

Moreover, the medical imaging system unit 1 comprises an execution unit If for completing the computing task T with the computing result CRₖ received from the selected remote computing unit 2.

FIG 12 shows a schematic view on a computing unit 2 for performing a sub-task Tₖ of a medical imaging system unit 1 according to an embodiment of the invention.

The computing unit 2 comprises a communication unit 2a for exchanging a sequence S-TDS of signals with the remote medical imaging system unit 1, preferably over a distribution unit 3 (not shown in FIG 12), whereby the sequence S-TDS of signals TDS comprises at least a request signal S1 received from the remote medical imaging system unit 1.

Moreover, the computing unit 2 comprises a reception unit 2b for receiving the sub-task Tₖ from the remote medical imaging system unit 1.

The computing unit 2 further comprises an execution unit 2c for executing the received sub-task Tₖ and generating a computing result CRₖ of the sub-task Tₖ.

Besides, the computing unit 2 comprises a transmission unit 2d for transmitting the computing result CRₖ, preferably via the distribution unit 3 (not shown in FIG 12) to the remote medical imaging system unit 1 (shown in FIG 11).

The above descriptions are merely preferred embodiments of the present disclosure, but not intended to limit the present disclosure, and any modifications, equivalent replacements, improvements, etc. made within the spirit and principle of the present disclosure should be included within the scope of protection of the present disclosure.

Further, the use of the undefined article "a" or "one" does not exclude that the referred features can also be present several times. Likewise, the term "unit" or "device" does not exclude that it consists of several components, which may also be spatially distributed.

## Claims

1. Method of performing a medical imaging process, comprising the steps of:
- dividing a computing task (T) of a medical imaging system unit (1) into a set (S) of sub-tasks (T₁, ..., Tₙ) , wherein the computing task (T) is related to a generation of medical image data based on measurement data,
- selecting at least one sub-task (Tₖ) of the set (S) of sub-tasks (T_{ι}, ..., Tₙ) for external execution,
- exchanging a sequence (S-TDS) of task delegation signals (TDS) with a plurality of remote computing units (2), preferably via a distribution (3) unit, for selecting at least one of the remote computing units (2) for execution of the selected at least one sub-task (Tₖ), whereby the sequence (S-TDS) of task delegation signals (TDS) comprises at least one request signal (RS) sent by the medical imaging system unit (1),
- outsourcing the at least one selected sub-task (Tₖ) to the selected remote computing unit (2) for remotely generating a computing result (CRₖ) of the at least one selected sub-task (Tₖ) and
- receiving the generated computing result (CRₖ) from the selected remote computing unit (2),
- completing the computing task (T) with the computing result (CRₖ) received from the selected remote computing unit (2).

2. Method according to claim 1, wherein the step of exchanging the sequence (S-TDS) of signals, including task delegation signals (TDS), includes the steps of:
- sending a first request signal (S1) to the plurality of remote computing units (2), preferably via the distribution unit (3),
- receiving a service proposal signal (S2) from at least one remote computing unit (2) of the plurality of remote computing units (2), preferably via the distribution unit (3),
- selecting at least one remote computing unit (2) of the at least one remote computing unit (2) for executing the selected sub-task (Tₖ) based on the received service proposal signal (S2),
- sending a second request signal (S3) for execution of the selected sub-task (Tₖ) to the selected remote computing unit (2),
- receiving a commitment signal (S4) of the at least one remote computing unit (2) for execution of the selected sub-task (Tₖ) .

3. Method according to claim 2, wherein the step of sending a second request signal (S3) comprises transmitting an offer to the selected remote computing unit (2) containing details of a criteria for a compensation of a remote execution of the selected sub-task (Tₖ) with a quantitatively defined access to resources.

4. Method according to claim 3, wherein the criteria includes details about the shape of the functional definition of an award function (A).

5. Method according to any of the claims 2 to 4, wherein the step of receiving a commitment signal (S4) comprises receiving a rejection or acceptance from the selected remote computing unit (2).

6. Method according to any of the claims 2 to 5, wherein the step of exchanging the sequence (S-TDS) of signals includes at least one of the sub-steps of:
- transmitting an answer signal (S5) to the selected remote computing unit (2) including an information of decline or commit for a compensation of a remote execution of the selected sub-task (Tₖ) with a quantitatively defined access to resources,
- receiving an answer signal (S6) to the answer signal (S5) to the selected remote computing unit (2) from the selected remote computing unit (2) including an information message including the computing result (CRₖ) of the selected sub-task (Tₖ) or no result,
- transmitting a compensation signal (S7), comprising a compensation of the remote execution of the selected sub-task (Tₖ) by a quantitatively defined access to resources to the selected remote computing unit (2).

7. Method according to any of the preceding claims, wherein the request signal (S1) comprises details about the sub-task (Tₖ) to be executed.

8. Method according to any of the claims 2 to 7, wherein the service proposal signal (S2) comprises an estimate time (Tₑ), by when the sub-task (Tₖ) can be executed.

9. Method according to claim 8, wherein the request signal (S1, S3) comprises an award value (A₀) if the sub-task (Tₖ) is executed at the estimate time (Tₑ) estimated by the selected remote computing unit (2), and a maximum award value (Aₘ) if the sub-task (Tₖ) is executed earlier.

10. Method according to any of the claims 6 to 9, wherein a compensation signal (S7) is transmitted to the selected remote computing unit (2) and the compensation signal (S7) comprises an award (A) for compensation of execution of the selected sub-task (Tₖ).

11. Method of performing a sub-task (Tₖ) of a remote medical imaging system unit (1), comprising the steps of:
- exchanging a sequence (S-TDS) of signals, including task-delegation signals (TDS), with the remote medical imaging system unit (1), preferably via a distribution unit (3), whereby the sequence (S-TDS) of signals comprises at least a request signal (S1) received from the remote medical imaging system unit (1),
- receiving the sub-task (Tₖ) from the remote medical imaging system unit (1),
- executing the received sub-task (Tₖ) and generating a computing result (CRₖ) of the sub-task (Tₖ),
- transmitting the computing result (CRₖ), preferably via the distribution unit (3), to the remote medical imaging system unit (1).

12. Method according to claim 11, wherein the step of exchanging a sequence (S-TDS) of signals includes at least one of the sub-steps of:
- receiving a first request signal (S1) from the medical imaging system unit (1), preferably via the distribution unit (3),
- sending a service proposal signal (S2), preferably via the distribution unit (3), to the medical imaging system unit (1)
- receiving a second request signal (S3) for execution of the selected sub-task (Tₖ) from the medical imaging system unit (1),
- sending a commitment signal (S4) for execution of the selected sub-task (Tₖ) to the medical imaging system unit (1) .

13. Method of distributed performing of a medical imaging process of a medical imaging system (10), whereby a medical imaging system unit (1) of the medical imaging system (10), which is responsible to execute a computing task (T) related to a medical imaging process, acts according to any of the preceding claims 1 to 9, and whereby at least one remote computing unit (2) acts according to any of the preceding claims 10 to 12, in order to execute the medical imaging process.

14. Medical imaging system unit (1), comprising:
- a division unit (la) for dividing a computing task (T) into a set (S) of sub-tasks (T₁, ..., Tₙ), wherein the computing task (T) is related to a generation of medical image data based on measurement data,
- a selection unit (lb) for selecting a sub-task (Tₖ) of the sub-tasks (T_{ι}, ..., Tₙ) for external execution,
- a communication unit (1c) for exchanging a sequence (S-TDS) of task delegation signals (TDS) with a plurality of remote computing units (2), preferably via a distribution unit (3), for selecting at least one of the remote computing units (2) for execution of the selected sub-task (Tₖ), whereby the sequence (S-TDS) of task delegation signals (TDS) comprises at least one request signal (RS) sent by the medical imaging system unit (1),
- an outsourcing unit (Id) for outsourcing the selected sub-task (Tₖ) to the selected remote computing unit (2) for remotely generating a computing result (CRₖ) of the selected sub-task (Tₖ) and
- a reception unit (Ie) for receiving the generated partial computing result (CRₖ) from the selected remote computing unit (2),
- an execution unit (If) for completing the computing task (T) with the computing result (CRₖ) received from the selected remote computing unit (2).

15. A computing unit (2) for performing a sub-task (Tₖ) of a remote medical imaging system unit (1), comprising:
- a communication unit (2a) for exchanging a sequence (S-TDS) of signals with the remote medical imaging system unit (1), preferably over a distribution unit (3), whereby the sequence (S-TDS) of signals comprises at least a request signal (S1) received from the remote medical imaging system unit (1),
- a reception unit (2b) for receiving the sub-task (Tₖ) from the remote medical imaging system unit (1),
- an execution unit (2c) for executing the received sub-task (Tₖ) and generating a computing result (CRₖ) of the sub-task (Tₖ),
- a transmission unit (2d) for transmitting the computing result (CRₖ), preferably via the distribution unit (3) to the remote medical imaging system unit (1).

16. Medical imaging network (10, 10', 10''), comprising:
- at least one medical imaging system unit (1) according to claim 14,
- at least one remote computing unit (2) according to claim 15,
- optionally a distribution unit (3) for conveying communication between the at least one medical imaging system unit (1) and the at least one remote computing unit (2).

17. Computer program product with a computer program, which can be loaded directly into a memory device of a medical imaging network (10, 10', 10''), with program sections to perform all the steps of the method according to any of the claims 1 to 12, when the computer program is carried out in the medical imaging network (10, 10', 10'').

18. Computer readable medium, on which program sections that can be read in and executed by a computer unit are stored in order to carry out all steps of the method according to any of the claims 1 to 12, when the program sections are executed by the computer unit.
